# FASCICULE DE BREVET EUROPEEN

(11) **EP 4 469 008 B1**
(45) Date de publication et mention de la délivrance du brevet: **31.12.2025**
(21) Numéro de dépôt: 23703511.8
(22) Date de dépôt: 13.01.2023
(51) Int. Cl.: A61F 2/44, A61F 2/30

(54) **CAGE APTE À ÊTRE IMPLANTÉE ENTRE DEUX PORTIONS OSSEUSES**
KÄFIG ZUR IMPLANTATION ZWISCHEN ZWEI KNOCHENTEILEN
CAGE SUITABLE FOR BEING IMPLANTED BETWEEN TWO BONE PORTIONS

(30) Priorité: 28.01.2022 FR 2200764
(43) Date de publication de la demande: 04.12.2024
(73) Titulaire: Razian, Hassan, 94240 L'Hay-les-Roses (FR)
(72) Inventeur: RAZIAN, Sam, 94240 L'HAY LES ROSES (FR); RAZIAN, Salar, 94240 L'HAY LES ROSES (FR)
(74) Mandataire: Boüan du Chef du Bos, Louis-Paterne
(86) Numéro de dépôt international: PCT/FR2023/000006
(87) Numéro de publication internationale: WO 2023/144475

(56) Documents cités:
- EP-A1- 0 986 350
- FR-A1- 2 943 529
- US-A1- 2013 268 076
- US-A1- 2014 277 473

## Description

### Domaine technique

La présente invention concerne les cages aptes à être implantées entre deux portions osseuses qui trouvent une application particulièrement avantageuse comme cages intersomatiques prévues pour être implantées par des chirurgiens entre deux vertèbres, notamment pour en faciliter leur implantation.

On connaît déjà des cages aptes à être interposées au contact entre deux portions osseuses, comme par exemple celle décrite et illustrée dans le FR2943529 ou US2013/268076A1.

Ces cages comprennent au moins au moins un corps solide délimité par deux faces d'appui opposées, chacune de ces faces d'appui étant assimilable à une surface plane, les deux surfaces planes étant sensiblement parallèles entre elles, ces deux faces d'appui étant en outre agencées pour être aptes à venir au contact respectivement des deux portions osseuses, ainsi que, généralement située dans une cavité réalisée dans ce corps, au moins une lame couplée à des moyens pour la faire pivoter de façon que, dans une position de repos elle soit entièrement contenue dans la cavité et que, suite à une rotation par rapport au corps, elle émerge au moins partiellement de cette cavité pour venir s'ancrer dans la ou les portions osseuses et assurer la fixation potentiellement définitive de la cage par rapport aux portions osseuses.

Une telle cage donne satisfaction dans la grande majorité des cas, mais peut présenter un inconvénient, à savoir le fait que, lorsque les os, comme des vertèbres, bougent, elle peut avoir tendance à migrer latéralement, ce qui peut avoir des conséquences négatives, et même dangereuses, pour le patient sur lequel elle a été implantée. Dans ce cas, le chirurgien peut être amené à intervenir pour repositionner la cage, et dans ce cas peut être confronté à un problème constitué par le fait que le corps de la cage est plus ou moins coincé pour différentes raisons qu'il n'est pas nécessaire d'expliciter ici car elles n'entrent pas dans le cadre de la présente invention.

Aussi, la présente invention a-t-elle pour but de réaliser une cage qui, tout en présentant une structure relativement simple et de réalisation peu onéreuse, tente de pallier l'inconvénient mentionné ci-dessus des cages similaires de l'art antérieur, et qui de plus peut faciliter le travail du chirurgien.

### Définition de l'invention

Plus précisément, la présente invention a pour objet une cage apte à être implantée entre deux portions osseuses; ladite cage comprenant au moins un corps solide délimité par deux faces d'appui opposées, chacune de ces faces d'appui étant assimilable à une surface plane, les deux surfaces planes étant sensiblement parallèles entre elles, ces deux faces d'appui opposées étant en outre agencées pour être aptes à venir au contact respectivement des deux portions osseuses, caractérisée par le fait qu'elle comporte en outre :
- une came définissant un axe longitudinal de came ;
- une lame définissant un axe longitudinal de lame,
- ladite came et ladite lame étant agencées de façon que, dans une position dite « de repos », elles soient entièrement contenues dans l'espace dit « interfacial » défini entre les deux plans respectivement des deux faces d'appui ; et
- des moyens pour commander le déplacement de ladite came et de ladite lame par rapport audit corps de façon que :
   * lors d'un premier déplacement par rapport à ladite position de repos, ladite came soit apte à émerger partiellement dudit espace interfacial par au moins l'une des deux faces d'appui, ladite lame restant entièrement contenue dans ce dit espace interfacial et que,
   * lors d'un second déplacement par rapport à ladite position de repos, ladite lame soit apte à émerger partiellement dudit espace interfacial par au moins l'une des deux faces d'appui, ladite came restant entièrement contenue dans ce dit espace interfacial.

### Brève Description des dessins

D'autres caractéristiques et avantages de la présente invention apparaîtront au cours de la description suivante donnée en regard des dessins annexés à titre illustratif, mais nullement limitatif, dans lesquels :
- la figure 1 est un schéma permettant de mettre en exergue les caractéristiques structurelles de la cage selon l'invention,
- la figure 2 est une vue partielle d'une cage selon l'invention dans une configuration définie comme position dite « de repos »,
- la figure 3 est une vue partielle de la cage selon l'invention en accord avec l'illustration selon la figure 2, dans une configuration définie comme position dite « de distraction »
- la figure 4 est une vue partielle de la cage selon l'invention en accord avec les illustrations selon les figures 2 et 3, dans une configuration définie comme position dite « d'implantation par lame »,
- la figure 5 est une vue en perspective cavalière d'une cage selon l'invention dans une réalisation industrielle et conforme aux illustrations selon les figures 1 à 4.

### Avertissements

Il est de plus précisé que, dans la présente description, si l'adverbe "sensiblement" est associé à un qualificatif d'un moyen donné, ce qualificatif doit être compris au sens strict ou approché.

### Description d'un mode de réalisation préféré de l'objet de l'invention.

La cage selon l'invention est apte à être implantées entre deux portions osseuses OS1, OS2, par exemple deux corps vertébraux consécutifs.

La cage selon l'invention comprend un corps solide 10 délimité par deux faces d'appui opposées 11-1, 11-2, chacune de ces faces d'appui étant assimilable à une surface plane, les deux surfaces planes étant sensiblement parallèles entre elles. Elles peuvent certes, dans certains cas, présenter une certaine angulation l'une par rapport à l'autres mais, cette angulation étant généralement très faible, elles sont malgré tout assimilables à deux faces parallèles.

Ces faces d'appui opposées 11-1, 11-2 sont agencées pour être aptes à venir respectivement au contact, de façon relativement congruente, des deux portions osseuses OS1, OS2.

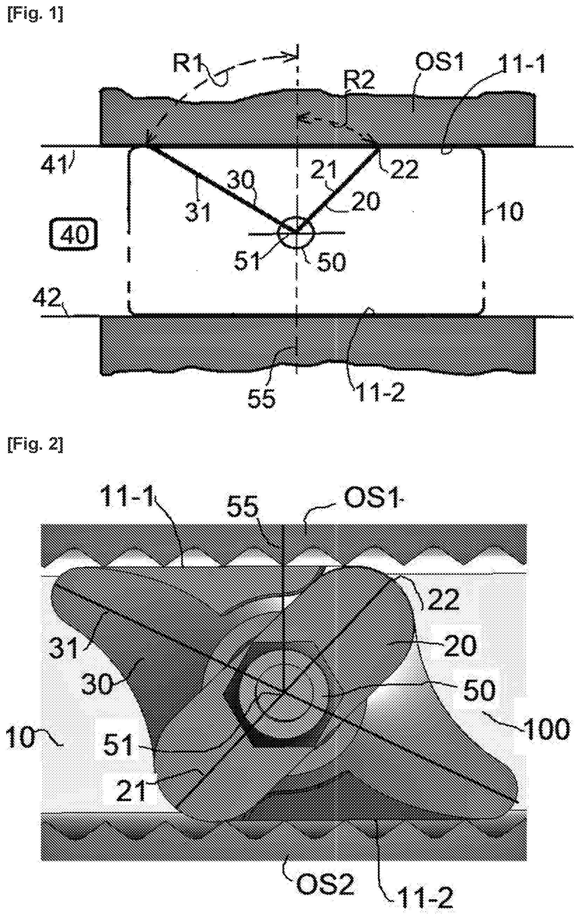

Selon une caractéristique de l'invention, la cage comporte en outre une came 20 définissant un axe longitudinal de came 21 et une lame 30 définissant un axe longitudinal de lame 31. La came 20 et la lame 30 sont agencées de façon que, dans une position dite « de repos » (figures 1 et 2), elles soient entièrement contenues dans l'espace 40 dit « interfacial » défini entre les deux plans 41, 42 respectivement des deux faces d'appui opposées 11-1, 11-2.

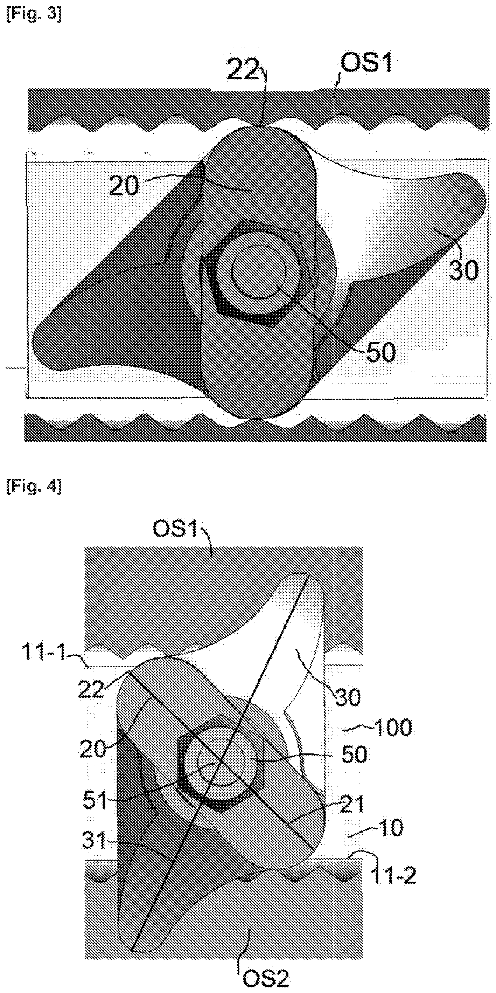

Selon une autre caractéristique de l'invention, la cage comporte des moyens pour commander le déplacement de la came 20 et de la lame 30 par rapport au corps 10 de façon que :
(i) lors d'un premier déplacement par rapport à la position de repos figure 2, la came 20 soit apte à émerger partiellement de l'espace interfacial par au moins l'une 11-1 des deux faces d'appui, la lame 30 restant entièrement contenue dans cet espace interfacial 40, la cage prenant alors, comme explicité ci-après, une configuration définie dans une position dite « de distraction » (figure 3), et que
(ii) lors d'un second déplacement par rapport à la position de repos, la lame 30 soit apte à émerger partiellement de l'espace interfacial 40 par au moins l'une 11-1 des deux faces d'appui, la came restant entièrement contenue dans cet espace interfacial 40, la cage prenant alors une configuration définie dans une position dite « d'implantation par lame » (figure 4).

Selon une caractéristique préférentielle avantageuse de l'invention, les moyens pour commander le déplacement de la came 20 et de la lame 30 par rapport au corps 10 sont constitués par un arbre 50 monté en rotation par rapport au corps 10 selon un axe de rotation 51 sensiblement parallèle à la au moins une face d'appui 11-1 selon les illustrations qui ne sont pas limitatives, et des moyens pour solidariser la came 20 et la lame 30 sur cet arbre 50 de façon qu'elles soient sensiblement contenues respectivement dans des plans faisant, avec l'axe de rotation 51, un angle non nul, optionnellement un angle droit.

Des moyens pour monter un arbre en rotation par rapport au corps 10, sont bien connus en eux-mêmes notamment dans le domaine des cages intervertébrales de l'art antérieur. Ils ne seront pas plus amplement décrits ici.

Quant aux moyens pour solidariser la came et la lame avec l'arbre, ils peuvent être de tout type, par exemple soudage, sertissage, vissage, clipsage, etc.

Selon une autre caractéristique avantageuse de l'invention, les axes longitudinaux 21, 31 respectivement de la came 20 et de la lame 30 sont situés respectivement de part et d'autre du plan axial 55 contenant l'axe de rotation 51 et sensiblement perpendiculaire aux deux faces d'appui 11-1, 11-2.

Les premier et second déplacements décrits ci-dessus de la came 20 et de la lame 30 par rapport au corps 10 sont respectivement deux rotations de l'arbre 50, en sens contraire R1, R2 l'une de l'autre, figure 1. Ces deux rotations de l'arbre 50 sont généralement commandées manuellement par le chirurgien, par exemple au moyen d'un ancillaire équivalent, quant à son fonctionnement, à un tournevis ou analogue.

Selon une autre caractéristique très préférentielle de l'invention, la longueur de la lame 30 prise selon son axe longitudinal 31 est supérieure à la longueur de la came 20 prise selon son axe longitudinal 21, l'extrémité libre 22 de la came 20 opposée à l'arbre de rotation 50 étant non pénétrante. De plus la longueur respectivement de la lame 30 et de la came 20 est supérieure à la distance séparant l'axe de rotation 50 de la au moins une 11-1 face d'appui.

De façon très préférentielle l'extrémité libre 22 (opposée à l'arbre de rotation 50) de la came 20 est de forme arrondie non pénétrante pour former une butée par frottement.

La cage illustrée sur la figure 1 comporte un couple d'une lame 30 et d'une came 20 qui sont conformées pour être situées chacune dans presque leur totalité du même côté de l'arbre de rotation 50.

Cependant, il est précisé que l'invention s'applique aussi à un double-couple lame/came, ces dernières présentant une symétrie axiale par rapport à l'axe de rotation 51, comme visible sur la cage qui est illustrée sur les figures 2 à 5.

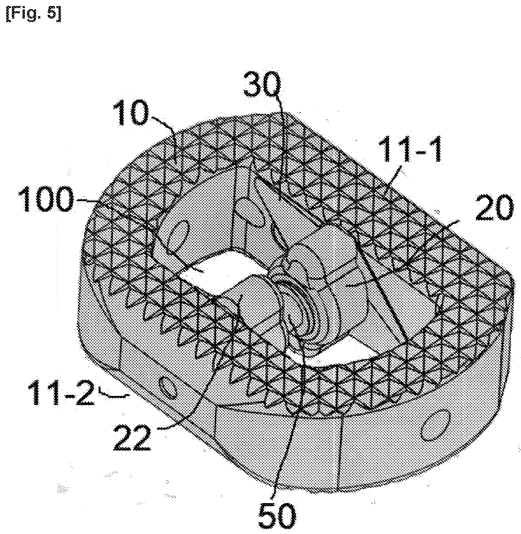

Il est même possible que la cage comporte plus d'un double-couple, à savoir par exemple deux doubles-couples comme illustré sur la figure 5.

De même : un tel couple lame/came peut-être situé dans une cavité 100 telle qu'une percée traversante réalisée dans le corps 10, comme illustré sur les figures. Mais il est possible qu'au moins l'un de ces couples lame/came soit situé sur un côté latéral du corps 10, bien que la structure de cage avec une cavité 100 contenant un tel couple lame/came soit la réalisation préférée.

A tire d'exemple de réalisation préférentielle, mais non limitatif : le corps 10 de la cage peut être réalisé en PEEK, à savoir en Polyétheréthercétone ; la lame 30 et la came 20 peuvent être réalisées en un alliage de titane du type TA6V-ELI à base d'aluminium-vanadium.

La cage selon l'invention fonctionne et s'utilise de la façon suivante décrite plus particulièrement en regard des figures 2 à 4 dans un cas possible pouvant se présenter à un Chirurgien.

Il est dans ce cas considéré que la cage est implantée entre deux vertèbres OS1 et OS2, se trouvant dans sa configuration d'implantation par lame, figure 4.

Le chirurgien peut estimer que l'implantation de la cage ne le satisfait pas et qu'il désire en modifier sa position. Mais il est possible qu'il se trouve devant le fait que la cage est bien « bloquée » entre les deux vertèbres (pour différentes raisons qu'il n'est pas nécessaire d'expliciter ici). Il se trouve donc confronté à des difficultés pour la déplacer et ajuster sa position.

Pour surmonter cet inconvénient dans le cas évoqué, il fait tout d'abord tourner R1 l'arbre 50 pour amener la cage dans sa position de repos (figure 2).

Le chirurgien fait alors pivoter R2 l'arbre 50 pour amener l'extrémité 22 (ou les deux extrémités opposées) de la came 20 au contact de la portion osseuse OS1 (ou respectivement sur les deux portions osseuses OS1 et OS2), puis continue la rotation R2 de l'arbre jusqu'à la position de distraction (figure 3). Dans ce mouvement de la came 20, celle-ci joue le rôle de levier pour repousser et donc détacher la face d'appui 11-1 (ou les deux faces d'appui 11-1 et 11-2) de la portion osseuse OS1 (ou des portions osseuses OS1, OS2). Il s'en suit que la cage est détachée et libérée de ces portions osseuses.

La cage est rendue plus facilement déplaçable après l'avoir ramenée à sa configuration de repos (figure 2).

Dans cette position de repos, le chirurgien peut facilement faire subir à la cage des déplacements, essentiellement dans le plan intervertébral, pour la positionner selon son souhait notamment en fonction de son diagnostic.

Une fois la cage repositionnée selon son souhait, il peut à nouveau commander la rotation de l'arbre 50 selon une rotation R1 pour faire pénétrer la lame dans la (les) portion(s) osseuse(s) pour lui faire prendre la position de fixation par lame (figure 4) et la solidariser alors plus définitivement.

Les caractéristiques structurelles de la cage selon l'invention soulignées ci-dessus permettent d'assurer un ancrage de la cage dans les deux portions d'os OS1, OS2 de façon encore plus parfaite que celle des cages de l'art antérieur, ce qui est primordial pour un patient, tout en facilitant le travail d'implantation d'une telle cage par le chirurgien.

## Revendications

1. Cage apte à faciliter son implantation entre deux portions osseuses (OS1, OS2), ladite cage comprenant au moins :
• un corps solide (10) délimité par deux faces d'appui opposées (11-1, 11-2), chacune de ces faces d'appui étant assimilable à une surface plane, ces deux surfaces planes étant sensiblement parallèles entre elles, ces deux faces d'appui étant en outre agencées pour être aptes à venir au contact respectivement des deux portions osseuses (OS1, OS2),
**caractérisée par le fait qu'**elle comporte en outre :
• une came (20) définissant un axe longitudinal de came (21) ;
• une lame (30) définissant un axe longitudinal de lame (31),
• ladite came (20) et ladite lame (30) étant agencées de façon que, dans une position dite « de repos », elles soient entièrement contenues dans l'espace (40) dit « interfacial » défini entre les deux plans (41, 42) respectivement des deux faces d'appui (11-1, 11-2) ;
• des moyens pour commander le déplacement de ladite came (20) et de ladite lame (30) par rapport audit corps (10) de façon que :
* lors d'un premier déplacement par rapport à ladite position de repos, ladite came (20) soit apte à émerger partiellement dudit espace interfacial par au moins l'une (11-1) des deux faces d'appui, ladite lame (30) restant entièrement contenue dans ce dit espace interfacial (40), et que
* lors d'un second déplacement par rapport à ladite position de repos, ladite lame (30) soit apte à émerger partiellement dudit espace interfacial (40) par au moins l'une (11-1) des deux faces d'appui, ladite came restant entièrement contenue dans ce dit espace interfacial (40).

2. Cage selon la revendication 1, **caractérisée par** le fait les moyens pour commander le déplacement de ladite came et de ladite lame par rapport audit corps sont constitués par :
• un arbre (50) monté en rotation par rapport audit corps (10) selon un axe de rotation (51) sensiblement parallèle à la au moins une face d'appui (11-1, 11-2), et
• des moyens pour solidariser ladite came (20) et de ladite lame (30) sur ledit arbre (50), de façon qu'elles soient sensiblement contenues respectivement dans des plans faisant, avec ledit axe de rotation (51), un angle non nul, optionnellement un angle droit.

3. Cage selon la revendication 2, **caractérisée par le fait que** les axes longitudinaux (21, 31) respectivement de la came (20) et de la lame (30) sont situés respectivement de part et d'autre du plan axial (55) contenant ledit axe de rotation (51) et sensiblement perpendiculaire aux deux faces d'appui (11-1, 11-2).

4. Cage selon la revendication 3, **caractérisée par le fait que** les premier et second déplacements de ladite came (20) et de ladite lame (30) par rapport audit corps (10) sont respectivement deux rotations dudit arbre (50), en sens contraire (R1, R2) l'une de l'autre.

5. Cage selon l'une des revendications précédentes, **caractérisée par le fait que** la longueur de ladite lame (30) prise selon son axe longitudinal (31) est supérieure à la longueur de ladite came (20) prise selon son axe longitudinal (21), l'extrémité libre (22) de ladite came (20) opposée à l'arbre de rotation (50) étant non pénétrante.

6. Cage selon la revendication 5 quand elle dépend de la revendication 2, **caractérisée par le fait que** la longueur respectivement de ladite lame (30) et de ladite came (20) est supérieure à la distance séparant ledit axe de rotation (50) et ladite au moins une (11-1) face d'appui.

7. Cage selon l'une des revendications 5 et 6, **caractérisée par le fait que** l'extrémité libre (22) de ladite came (20) opposée à l'arbre de rotation (50) non pénétrante est de forme arrondie formant butée par frottement.

## Patentansprüche

1. Gestell, das zum Ermöglichen seiner Implantation zwischen zwei Knochenabschnitten (OS1, OS2) geeignet ist, das Gestell umfassend mindestens:
• einen Festkörper (10), der durch zwei gegenüberliegenden Auflageflächen (11-1, 11-2) begrenzt wird, wobei jede dieser Auflageflächen mit einer ebenen Oberfläche vergleichbar ist, wobei diese zwei ebenen Oberflächen im Wesentlichen parallel zueinander sind, wobei diese zwei Auflageflächen ferner angeordnet sind, um dazu geeignet zu sein, mit jeweils den zwei Knochenabschnitten (OS1, OS2) in Kontakt zu kommen, **gekennzeichnet durch die Tatsache, dass** es ferner vorweist:
• eine Nocke (20), die eine Nockenlängsachse (21) definiert;
• eine Klinge (30), die eine Klingenlängsachse (31) definiert,
• wobei die Nocke (20) und die Klinge (30) angeordnet sind, sodass sie in einer Position, der sogenannten "Ruheposition", vollständig in dem Raum (40), dem sogenannten "Grenzflächenraum" enthalten sind, der zwischen den zwei Ebenen (41,42) der zwei Auflageflächen (11-1, 11-2) definiert ist;
• Mittel zum Steuern der Bewegung der Nocke (20) und der Klinge (30) relativ zu dem Körper (10), sodass:
* bei einer ersten Bewegung relativ zu der Ruheposition, die Nocke (20) zum teilweisen Herausragen aus dem Grenzflächenraum durch mindestens eine (11-1) der zwei Auflageflächen geeignet ist, wobei die Klinge (30) in diesem sogenannten Grenzflächenraum (40) vollständig enthalten verbleibt, und dass
* bei einer zweiten Bewegung relativ zu der Ruheposition, die Klinge (30) zum teilweisen Herausragen aus dem Grenzflächenraum (40) durch mindestens eine (11-1) der zwei Auflageflächen geeignet ist, wobei die Nocke in diesem sogenannten Grenzflächenraum (40) vollständig enthalten verbleibt.

2. Gestell nach Anspruch 1, **gekennzeichnet durch die Tatsache, dass** die Mittel zum Steuern der Bewegung der Nocke und der Klinge relativ zu dem Körper bestehen aus:
• einer Welle (50), die relativ zu dem Körper (10) um eine Drehachse (51) drehbar montiert ist, die im Wesentlichen parallel zu mindestens einer Auflagefläche (11-1, 11-2) verläuft, und
• Mittel zum Anschließen der Nocke (20) und der Klinge (30) auf der Welle (50), sodass sie im Wesentlichen jeweils in Ebenen enthalten sind, die mit der Drehachse (51) einen Winkel ungleich Null, gegebenenfalls einen rechten Winkel, bilden.

3. Gestell nach Anspruch 2, **gekennzeichnet durch die Tatsache, dass** die Längsachsen (21,31)jeweils der Nocke (20) und der Klinge (30) jeweils auf beiden Seiten der axialen Ebene (55) gelegen sind, die die Drehachse (51) enthält, und im Wesentlichen senkrecht zu den zwei Auflageflächen (11-1, 11-2) ist.

4. Gestell nach Anspruch 3, **gekennzeichnet durch die Tatsache, dass** die erste und die zweite Bewegung der Nocke (20) und der Klinge (30) relativ zu dem Körper (10) jeweils zwei Umdrehungen der Welle (50) in entgegengesetzter Richtung (R1, R2) zueinander sind.

5. Gestell nach einem der vorstehenden Ansprüche, **gekennzeichnet durch die Tatsache, dass** die Länge der Klinge (30), genommen entlang ihrer Längsachse (31), größer als die Länge des Nockens (20), genommen entlang seiner Längsachse (21) ist, wobei das freie Ende (22) des Nockens (20), das der Drehwelle (50) gegenüberliegt, nicht durchdringend ist.

6. Gestell nach Anspruch 5, wenn er von Anspruch 2 abhängt, **gekennzeichnet durch die Tatsache, dass** die jeweilige Länge der Klinge (30) und der Nocke (20) größer als der Abstand ist, der die Drehachse (50) und die mindestens eine Auflagefläche (11-1) trennt.

7. Gestell nach einem der Ansprüche 5 und 6, **gekennzeichnet durch die Tatsache, dass** das freie Ende (22) des Nockens (20), das der nicht durchdringenden Drehwelle (50) gegenüberliegt, von abgerundeter Form ist und einen Anschlag durch Reibung ausbildet.

## Claims

1. Cage which is able to facilitate the implantation thereof between two bone portions (OS1, OS2), said cage comprising at least:
• a solid body (10) delimited by two opposite bearing faces (11-1, 11-2), each of these bearing faces being comparable to a plane surface, these two plane surfaces being substantially parallel to each other, these two bearing faces further being arranged to be able to come into contact with the two respective bone portions (OS1, OS2),
**characterized in that** said cage further comprises:
• a cam (20) defining a longitudinal cam axis (21);
• a blade (30) defining a longitudinal blade axis (31),
• said cam (20) and said blade (30) being arranged such that, in a "rest" position, they are entirely contained in the "interfacial" space (40) defined between the two respective planes (41, 42) of the two bearing faces (11-1, 11-2);
• means for controlling the movement of said cam (20) and said blade (30) relative to said body (10) such that:
* during a first movement relative to said rest position, said cam (20) is able to partially emerge from said interfacial space via at least one (11-1) of the two bearing faces, said blade (30) remaining entirely contained in said interfacial space (40), and that
* during a second movement relative to said rest position, said blade (30) is able to partially emerge from said interfacial space (40) via at least one (11-1) of the two bearing faces, said cam remaining entirely contained in said interfacial space (40).

2. Cage according to claim 1, **characterized in that** the means for controlling the movement of said cam and said blade relative to said body are made up of:
• a shaft (50) mounted for rotation relative to said body (10) about a rotation axis (51) which is substantially parallel to the at least one bearing face (11-1, 11-2), and
• means for securing said cam (20) and said blade (30) to said shaft (50), such that they are substantially contained in respective planes which form, with said rotation axis (51), a non-zero angle, optionally a right angle.

3. Cage according to claim 2, **characterized in that** the respective longitudinal axes (21, 31) of the cam (20) and the blade (30) are located on either side of the axial plane (55) which contains said rotation axis (51) and is substantially perpendicular to the two bearing faces (11-1, 11-2).

4. Cage according to claim 3, **characterized in that** the first and the second movement of said cam (20) and said blade (30) relative to said body (10) are two respective rotations of said shaft (50) in opposite directions (R1, R2) to each other.

5. Cage according to any of the preceding claims, **characterized in that** the length of said blade (30) taken along its longitudinal axis (31) is greater than the length of said cam (20) taken along its longitudinal axis (21), the free end (22) of said cam (20) opposite the rotation shaft (50) being non-penetrating.

6. Cage according to claim 5 when dependent on claim 2,
**characterized in that** the respective lengths of said blade (30) and said cam (20) are greater than the distance separating said rotation axis (50) from said at least one bearing face (11-1).

7. Cage according to either of claims 5 and 6, **characterized in that** the free end (22) of said cam (20) opposite the non-penetrating rotation shaft (50) has a rounded shape which forms a friction stop.
